# EUROPEAN PATENT APPLICATION

(11) **EP 3 875 867 A1**
(43) Date of publication of application: **08.09.2021**
(21) Application number: 19879177.4
(22) Date of filing: 25.10.2019
(51) Int. Cl.: F24F 11/80, F24F 7/007, F24F 110/10, F24F 110/70

(54) **ENVIRONMENTAL CONTROL SYSTEM AND ENVIRONMENTAL CONTROL METHOD**

(30) Priority: 02.11.2018 JP 2018207582
(71) Applicant: Panasonic Intellectual Property Management Co., Ltd., Osaka-shi, Osaka 540-6207 (JP)
(72) Inventor: AOKI, Saki, Osaka-shi, Osaka 540-6207 (JP); SUZUKA, Yuko, Osaka-shi, Osaka 540-6207 (JP); IWAHORI, Yutaka, Osaka-shi, Osaka 540-6207 (JP); KOSHIMIZU, Takanori, Osaka-shi, Osaka 540-6207 (JP)
(74) Representative: Appelt, Christian W.
(86) International application number: PCT/JP2019/041856
(87) International publication number: WO 2020/090640

(57) **Abstract**

An environmental control system (10) includes: an air conditioner (30) for adjusting a temperature in a space (300) in which a subject (200) is located; and a control apparatus (120) which performs control that makes a parasympathetic nervous system of the subject (200) dominant over a parasympathetic nervous system of the subject (200) by performing first control and second control using the air conditioner (30). The first control increases the temperature in the space (300), and the second control cyclically changes the temperature in the space (300) in such a manner that a temperature difference from a largest value to a smallest value in the space (300) falls within 3 degrees Celsius.

## Description

### [Technical Field]

The present invention relates to an environmental control system and an environmental control method.

### [Background Art]

Patent Literature 1 discloses an environmental control apparatus which detects physical states of a resident by using both biological information and behavior information, and controls housing equipment most appropriately for the individual based on the physical states.

### [Citation List]

### [Patent Literature]

[PTL 1] Japanese Unexamined Patent Application Publication No. 2001-041531

### [Summary of Invention]

### [Technical Problem]

The present invention provides environmental control systems and environmental control methods which make it possible to, for example, reduce a feeling of nervousness of a subject and increase a feeling of relaxing of the subject.

### [Solution to Problem]

An environmental control system according to an aspect of the present invention includes: an air conditioner for adjusting a temperature in a space in which a subject is located; and a control apparatus which performs control that makes a parasympathetic nervous system of the subject dominant over a parasympathetic nervous system of the subject by performing first control and second control using the air conditioner. The first control increases the temperature in the space, and the second control cyclically changes the temperature in the space in such a manner that a temperature difference from a largest value to a smallest value in the space falls within 3 degrees Celsius.

An environmental control system according to an aspect of the present invention includes: a ventilator which ventilates a space in which a subject is located; and a control apparatus which performs control that makes a parasympathetic nervous system of the subject dominant over a sympathetic nervous system of the subject by setting a concentration of carbon dioxide in the space to 1000 ppm or more using the ventilator.

An environmental control method according to an aspect of the present invention includes: performing control that makes a parasympathetic nervous system of the subject dominant over a parasympathetic nervous system of the subject using an air conditioner for adjusting a temperature in a space in which a subject is located, by increasing the temperature in the space and cyclically changing the temperature in the space in such a manner that a temperature difference from a largest value to a smallest value in the space falls within 3 degrees Celsius.

An environmental control method according to an aspect of the present invention includes: performing control that makes a parasympathetic nervous system of the subject dominant over a sympathetic nervous system of the subject by setting a concentration of carbon dioxide in the space to 1000 ppm or more using a ventilator which ventilates a space in which a subject is located.

### [Advantageous Effects of Invention]

According to the present invention, it is possible to implement the environmental control systems and the environmental control methods which make it possible to, for example, reduce a feeling of nervousness of a subject and increase a feeling of relaxing of the subject.

### [Brief Description of Drawings]

[FIG. 1] FIG. 1 is a diagram illustrating a configuration of the environmental control system according to the embodiment.
[FIG. 2] FIG. 2 is a block diagram illustrating a functional configuration of a control apparatus.
[FIG. 3] FIG. 3 is a time chart for explaining control which makes the sympathetic nervous system of a subject dominant.
[FIG. 4] FIG. 4 is a diagram illustrating one example of fluctuation of illuminance of light.
[FIG. 5] FIG. 5 is a diagram illustrating a function of sympathetic nervous system and a function of parasympathetic nervous system.
[FIG. 6] FIG. 6 is a flow chart of operation example 1 of an environmental control system according to an embodiment.
[FIG. 7] FIG. 7 is a diagram illustrating a relationship between the functions of the sympathetic nervous system (or the parasympathetic nervous system) and change in biological information.
[FIG. 8] FIG. 8 is a diagram for explaining control based on heart rates.

### [Description of Embodiments]

Hereinafter, embodiments according to the present disclosure are described with reference to the drawings. It is to be noted that each of the embodiments described below indicates a general or specific example. The numerical values, shapes, materials, constituent elements, the arrangement and connection of the constituent elements, etc. indicated in the following embodiments are mere examples, and do not limit the scope of the present invention. Among the constituent elements in the following embodiments, constituent elements not recited in the independent claim that defines the most generic concept of the present disclosure are described as optional constituent elements.

It is to be noted that each of the drawings is a schematic diagram, and is not necessarily illustrated precisely. In addition, in each of the drawings, substantially the same constituent elements may be assigned with the same numerical signs, and overlapping descriptions may be omitted or simplified.

### [Embod iment]

### [A Configuration of an Environmental Control System]

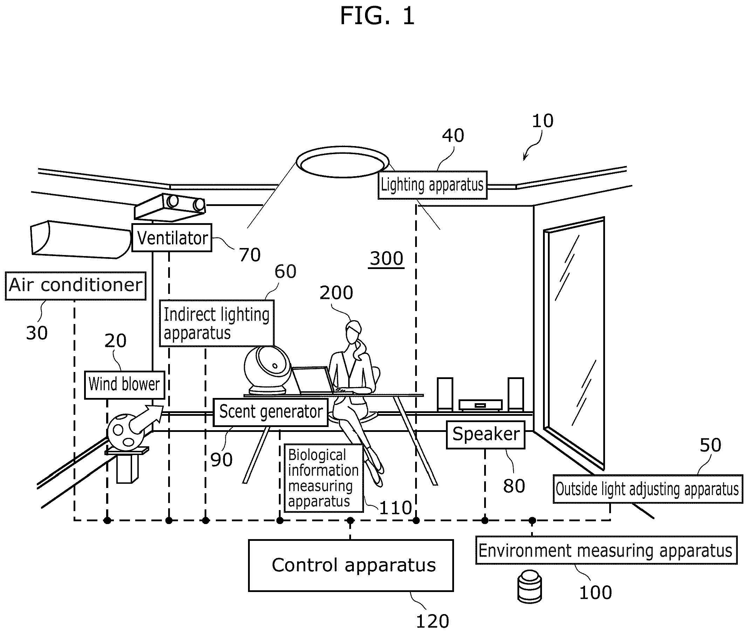

First, a configuration of an environmental control system according to an embodiment is described. FIG. 1 is a diagram illustrating the configuration of the environmental control system according to the embodiment.

Environmental control system 10 illustrated in FIG. 1 performs control for adjusting the function of the autonomic nervous system of subject 200 by controlling target apparatuses related to an environment in space 300 which is a closed space such as a room.

The autonomic nervous system of a human consists of two kinds of nervous systems that are a sympathetic nervous system and a parasympathetic nervous system which function in contrast. Environmental control system 10 performs control that makes the parasympathetic nervous system dominant over the sympathetic nervous system (the control is hereinafter also simply referred to as control making the parasympathetic nervous system dominant). Making the parasympathetic nervous system dominant over the sympathetic nervous system makes it possible to, for example, reduce the feeling of nervousness of subject 200 and increase the feeling of relaxing of subject 200.

Specifically, environmental control system 10 includes wind blower 20, air conditioner 30, lighting apparatus 40, outside light adjusting apparatus 50, indirect lighting apparatus 60, ventilator 70, speaker 80, scent generator 90, environment measuring apparatus 100, biological information measuring apparatus 110, and control apparatus 120.

Wind blower 20 is an apparatus which blows wind toward subject 200. Specifically, wind blower 20 is a wind blower which has a comparatively high directivity such as a circulator, and may be a fan.

Air conditioner 30 is an apparatus for adjusting a temperature in space 300 in which subject 200 is located. Air conditioner 30 is capable of adjusting a humidity in space 300. Air conditioner 30 makes the temperature and the humidity in space 300 closer to a temperature and a humidity directed by control apparatus 120.

Lighting apparatus 40 is an apparatus for direct lighting which illuminates space 300 in which subject 200 is located. Lighting apparatus 40 is, for example, a ceiling light including a light emitting element such as an LED as a light source. Lighting apparatus 40 may be another lighting apparatus such as a base light or a down light. Lighting apparatus 40 is capable of being subjected to light adjustment and color adjustment by control apparatus 120.

Outside light adjusting apparatus 50 is an apparatus which adjusts the amount of light that enters space 300 in which subject 200 is located. Outside light adjusting apparatus 50 is, for example, an electronic blind which can be implemented in the form of a light adjusting film. Outside light adjusting apparatus 50 may be electric blinds (electric shutters) or the like.

Indirect lighting apparatus 60 is an apparatus for indirect lighting disposed in space 300 in which subject 200 is located. In other words, indirect lighting apparatus 60 illuminates one or more structures such as walls, a ceiling, or the like which define space 300. For example, indirect lighting apparatus 60 is capable of changing emission colors by including a plurality of light sources which provide different emission colors. Indirect lighting apparatus 60 may provide optional emission colors by combining any of light sources and optical filters. The emission colors of indirect lighting apparatus 60 can be changed to any of a monochromatic red light, a monochromatic green light, and a monochromatic blue light. It is to be noted that the color of light that is emitted by indirect lighting apparatus 60 is not particularly limited, and may be an optional color according to tastes of a user.

Ventilator 70 ventilates space 300 in which subject 200 is located. Ventilator 70 does not have a temperature adjusting function, unlike air conditioner 30. Ventilator 70 is, for example, an Energy Recovery Ventilator (ERV). Ventilator 70 may be a ventilator which does not perform heat exchange such as a ventilation fan. Alternatively, ventilator 70 may be an open/close apparatus of a window installed in space 300.

Speaker 80 is an apparatus which is disposed in space 300 in which subject 200 is located, and outputs speech, music, or the like.

Scent generator 90 is an apparatus which is disposed in space 300 in which subject 200 is located, and generates a scent. Scent generator 90 is, for example, an aroma diffuser, and may be a generator which generates another scent.

Environment measuring apparatus 100 is an apparatus which measures environmental information in space 300 in which subject 200 is located. Environment measuring apparatus 100 is, for example, a temperature sensor which measures temperature in space 300, a humidity sensor which measures humidity in space 300, an illuminance sensor which measures illuminance in space 300, a CO₂ sensor which measures the concentration of carbon dioxide (CO₂) in space 300, or the like.

Biological information measuring apparatus 110 is an apparatus which measures biological information about subject 200. Biological information measuring apparatus 110 measures, as biological information, a body temperature, a blood pressure, a heart rate, a pulse wave, the amount of sweating, an epidermis temperature, a facial expression, etc. of subject 200Biological information measuring apparatus 110 may measure a Very Low Frequency (VLF), a High Frequency (HF), a Low Frequency (LF), inspiration time, exhaustion time, pause time, etc. which are calculated based on the heart rate, the pulse wave, and a respiratory variation waveform. Biological information measuring apparatus 110 is, for example, a wearable sensor (that is, a contact sensor) which is attached to the body of subject 200, and may be a non-contact sensor. Examples of such a non-contact sensor includes a radio wave sensor capable of measuring heart rates, respiratory rates, pulse waves, etc. and a camera capable of measuring pupil diameters or facial expressions.

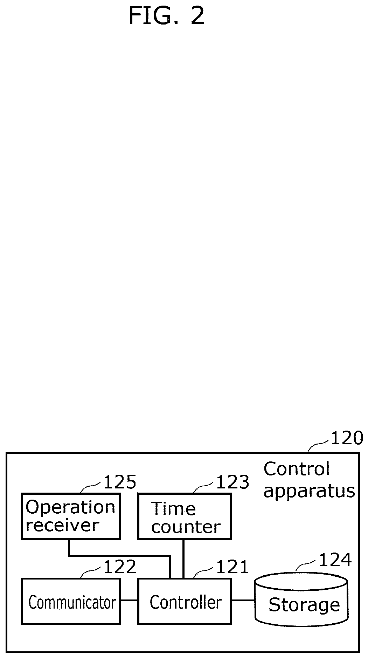

Control apparatus 120 is an apparatus which controls target apparatuses such as wind blower 20, air conditioner 30, lighting apparatus 40, outside light adjusting apparatus 50, indirect lighting apparatus 60, ventilator 70, speaker 80, and scent generator 90. FIG. 2 is a block diagram illustrating a functional configuration of control apparatus 120.

As illustrated in FIG. 2, control apparatus 120 includes controller 121, communicator 122, time counter 123, storage 124, and operation receiver 125.

Controller 121 controls target apparatuses by causing communicator 122 to transmit control signals. Controller 121 may be implemented in the form of, for example, a microcomputer, but may be implemented in the form of a processor.

Communicator 122 is a communication circuit (in other words, a communication module) which allows control apparatus 120 to communicate with the target apparatuses. For example, communicator 122 transmits control signals to target apparatuses under control of controller 121. In addition, communicator 122 receives environmental information about space 300 from environment measuring apparatus 100, and receives biological information of subject 200 from biological information measuring apparatus 110. Communicator 122 may perform wireless communication for example, but may perform wired communication. Communication standards for communication that is performed by communicator 122 are not particularly limited.

Time counter 123 measures current time. Time counter 123 is implemented in the form of a real time clock for example.

Storage 124 is a storage apparatus in which a control program allowing controller 121 to control each target apparatus is stored. Storage 124 is implemented in the form of a semiconductor memory for example.

Operation receiver 125 receives, from a user such as subject 200, an operation (for example, a setting operation regarding control that makes the parasympathetic nervous system dominant). Operation receiver 125 is implemented in the form of a touch panel, hardware buttons, or the like.

### [Control on the Air Conditioner]

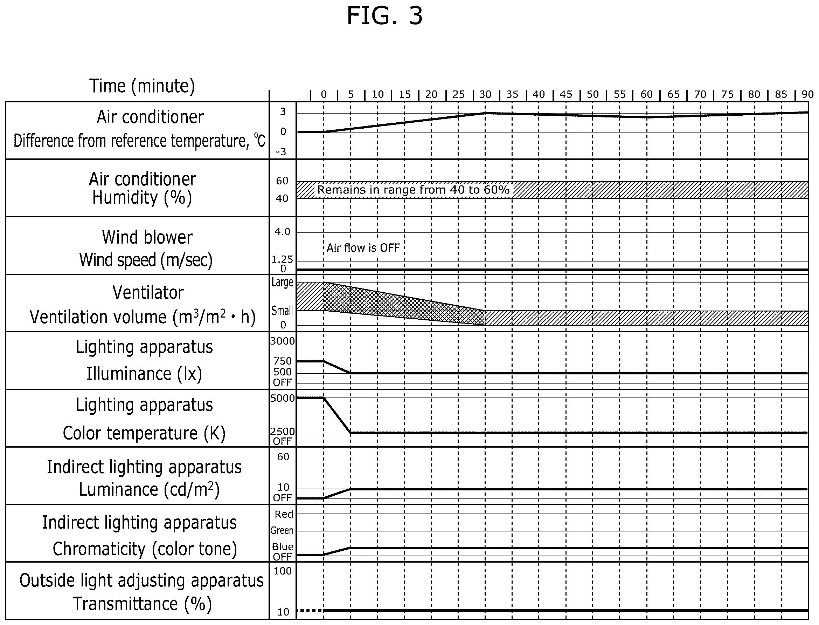

As described above, environmental control system 10 is capable of performing the control making the parasympathetic nervous system of subject 200 dominant. Hereinafter, details of the control are described. FIG. 3 is a time chart for explaining the control making the parasympathetic nervous system of subject 200 dominant. It is assumed that, in FIG. 3, the start time of the control is 0 minute, and as one example, target apparatuses are being controlled with consideration of only comfortableness before the start time. Although a period from 0 minute to 90 minutes is illustrated in FIG. 3, the same control as performed in the period from 0 minute to 90 minutes is repeated after the period.

It is to be noted that, in the control making the parasympathetic nervous system dominant, it is only necessary that at least one of the target apparatuses be controlled, but two or more of the target apparatuses may be controlled. This can make the parasympathetic nervous system of subject 200 to further dominant.

First, control on air conditioner 30 is described. Controller 121 of control apparatus 120 increases a temperature in space 300 using air conditioner 30 at an initial period from a start of the control making the parasympathetic nervous system dominant. Specifically, controller 121 increases the surrounding temperature from a reference temperature by 3 degrees Celsius for 30 minutes immediately after the start of the control. It is to be noted that the time (30 minutes) required to increase the temperature is one example, and is not particularly limited. It is desirable that the surrounding temperature be increased by approximately 3 degrees Celsius for 30 minutes.

Providing an environment in which a temperature in space 300 is slightly warmer than a reference temperature makes the parasympathetic nervous system of subject 200 dominant. Reference temperatures differ depending on seasons. For example, reference temperatures are a predetermined temperature in a range from 25 degrees Celsius to 27 degrees Celsius in summer, a predetermined temperature in a range from 21 degrees Celsius to 23 degrees Celsius in spring and autumn, and a predetermined temperature in a range from 17 degrees Celsius to 20 degrees Celsius in winter.

Controller 121 subsequently decreases the surrounding temperature by 1 degree Celsius for 30 minutes, and then increases the surrounding temperature by 1 degree Celsius for 30 minutes. After the above changes, such temperature changes are repeated. In other words, controller 121 cyclically changes the temperature in space 300 using air conditioner 30 in such a manner that the temperature difference from a largest value to a smallest value falls within 3 degrees Celsius. Surrounding temperatures around subject 200 are measured by, for example, environment measuring apparatus 100. Controller 121 controls air conditioner 30 based on the surrounding temperatures measured by environment measuring apparatus 100. Controller 121 may increase and decrease a temperature to be set of air conditioner 30 at the 60-minute cycle as described above without using environment measuring apparatus 100. It is to be noted that the cycle (60-minute cycle) for changing the temperatures is one example, and is not limited particularly.

In this way, controller 121 increases and decreases the temperature slightly (specifically, by approximately 3 degrees Celsius or less) at the predetermined cycle (for example, at the 60-minute cycle). In general, a human repeats basal metabolism in which his/her body is cooled down by sweating when the body generates heat excessively, and his/her body generates heat again after an elapse of time. Keeping a surrounding temperature around subject 200 constant leads to ignoring such a basal metabolism, and thus subject 200 inevitably feels too hot or too cold.

In comparison, slightly changing temperatures at a cycle with consideration of the body metabolism of subject 200 can increase comfortableness while reducing stimuli by temperatures given to subject 200. This can make the parasympathetic nervous system of subject 200 dominant.

Controller 121 keeps a humidity in space 300 in a range from 40% to 60% using air conditioner 30 during the control making the parasympathetic nervous system dominant.

### [Control on the Ventilator]

Next, control on ventilator 70 is described. Controller 121 sets the concentration of carbon dioxide in space 300 to 1000 ppm or more using ventilator 70. 1000 ppm is one example of a predetermined concentration. For example, controller 121 sets the concentration of carbon dioxide in space 300 to 1000 ppm or more by decreasing the ventilation volume of ventilator 70 when the concentration of the carbon dioxide in space 300 is low. Controller 121 may set the concentration of the carbon dioxide in space 300 to 1000 ppm or more by stopping ventilator 70. For example, the concentration of the carbon dioxide in space 300 is measured by environment measuring apparatus 100, and controller 121 controls ventilator 70 based on the concentration of the carbon dioxide measured by environment measuring apparatus 100.

Adjusting the concentration of the carbon dioxide in space 300 to 1000 ppm or more in this way can make the parasympathetic nervous system of subject 200 dominant.

### [Control on the Other Target Apparatuses]

Hereinafter, control on the other target apparatus is described while further referring to FIG. 3.

First, control on wind blower 20 is described. Controller 121 stops the wind blown by wind blower 20 in the control making the parasympathetic nervous system dominant. Although wind blower 20 is stopped before the start of the control in the example in FIG. 3, control of stopping wind blower 20 is performed if wind blower 20 is operating before the start of control.

Since this reduces stimuli given to subject 200 by the wind blown by wind blower 20, it becomes possible to make the parasympathetic nervous system dominant. It is to be noted that control for decreasing a wind speed more than the wind speed before the start of the control may be performed when wind blower 20 is operating before the start of the control. Even such control can make the parasympathetic nervous system dominant.

Next, control on lighting apparatus 40 is described. Controller 121 decreases an illuminance in space 300 using lighting apparatus 40 in the initial period from the start of the second control making the parasympathetic nervous system dominant. For example, controller 121 decreases the illuminance that is initially 750 lx in space 300 down to 500 lx for 5 minutes from the start of the control. It is desirable that the illuminance be changed with time in such a manner that subject 200 does not feel uncomfortable due to illuminance changes. "Decreasing the illuminance of lighting apparatus 40 in space 300" includes turning off lighting apparatus 40.

Reducing the illuminance in space 30 in this way reduces the stimuli by light given to subject 200, which can make the parasympathetic nervous system of subject 200 dominant.

In addition, controller 121 decreases the color temperature of the light emitted by lighting apparatus 40 in the initial period from the start of the control making the parasympathetic nervous system dominant. It is desirable that the chromaticity be changed with time in such a manner that subject 200 does not feel uncomfortable due to chromaticity changes. In the example in FIG. 3, the color temperature that was 5000 K initially of light emitted by lighting apparatus 40 is decreased to 2500 K.

In this way, providing space 300 with an illuminance environment in which the color temperature is low can make the parasympathetic nervous system of subject 200 dominant.

Next, control on outside light adjusting apparatus 50 is described. Controller 121 decreases a light amount of outside light that enters space 300 using outside light adjusting apparatus 50 in the initial period from the start of the control making the parasympathetic nervous system dominant. Specifically, controller 121 decreases a light transmittance of outside light adjusting apparatus 50. It is desirable that the illuminance be changed with time in such a manner that subject 200 does not feel uncomfortable due to illuminance changes. "Decreasing light transmittance of outside light adjusting apparatus 50" includes setting a transmittance rate of outside light adjusting apparatus 50 to 0% to block light.

Reducing the illuminance in space 30 in this way reduces the stimuli by light given to subject 200, which can make the parasympathetic nervous system of subject 200 dominant.

Next, control on indirect lighting apparatus 60 is described. Controller 121 changes an emission color of light to be emitted by indirect lighting apparatus 60 in the control making the parasympathetic nervous system dominant. For example, controller 121 increases a luminance (substantially, an illuminance) from 0 (colorless) cd/m² to 10 cd/m² for 5 minutes from the start of the control. The emission color at this time is blue for example, but may be emerald green, or another color. It is only necessary that controller 121 changes the chromaticity of light to be emitted by indirect lighting apparatus 60 in such a manner that the x-coordinate in a chromaticity diagram of a CIE 1931 color space of the chromaticity decreases in an initial period from a start of the control making the parasympathetic nervous system dominant. This can make the parasympathetic nervous system of subject 200 dominant.

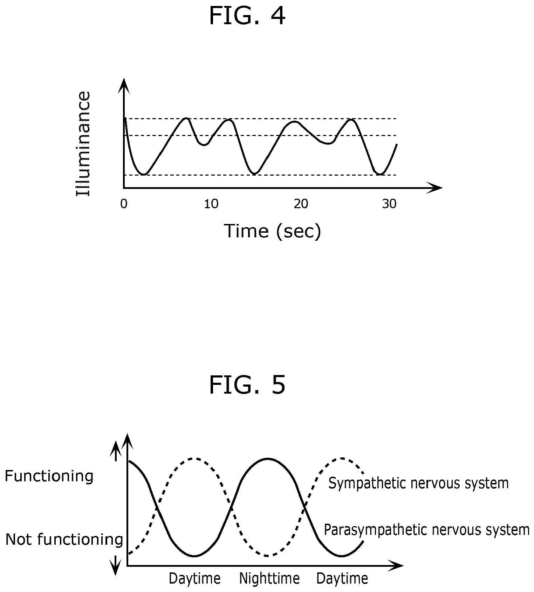

Controller 121 fluctuates the illuminance of the light to be emitted by indirect lighting apparatus 60 although the control is not precisely illustrated in FIG. 3. In other words, the brightness of the light to be emitted by indirect lighting apparatus 60 is increased or decreased at a cycle on the order of several seconds that is in a range approximately from 1 second to 10 seconds (a constant cycle or random cycles are possible). The light to be emitted by indirect lighting apparatus 60 may fluctuate with constant amplification or with random amplification. FIG. 4 is a diagram illustrating one example of fluctuation of illuminance of light. This can make the parasympathetic nervous system of subject 200 dominant.

Next, control on speaker 80 and scent generator 90 is described. Controller 121 changes a sound to be output by speaker 80 in the control making the parasympathetic nervous system dominant. "Changing a sound" here includes starting to output a sound in a state in which no sound is output. For example, speaker 80 outputs a sound which provides a relaxing effect such as a healing musical piece or a comparatively slow-tempo musical piece. This can make the parasympathetic nervous system of subject 200 dominant.

Controller 121 changes a scent to be generated by scent generator 90 in the control making the parasympathetic nervous system dominant. "Changing a scent" here includes starting to generate a scent in a state in which no scent is generated. Scent generator 90 generates a low-stimulus scent in which phytoncide, etc. is included, a scent of lavender, or the like. This can make the parasympathetic nervous system of subject 200 dominant.

### [Operation Example 1]

In general, in the human autonomic nervous system, the sympathetic nervous system is dominant over the parasympathetic nervous system in daytime, and the parasympathetic nervous system is dominant over the sympathetic nervous system in nighttime. FIG. 5 is a diagram illustrating the function of the sympathetic nervous system and the function of the parasympathetic nervous system. In other words, it can be said that a time zone in which the parasympathetic nervous system should be made dominant is roughly determined.

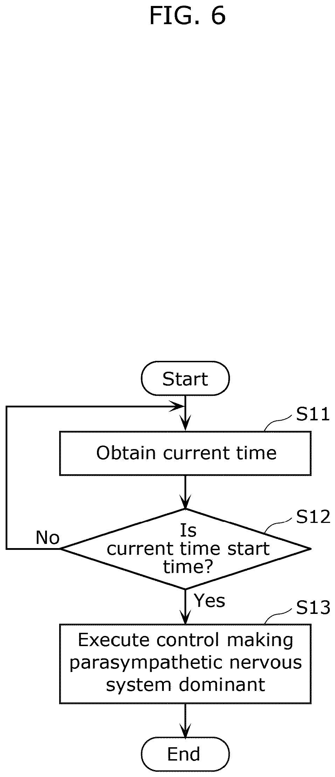

In view of this, control apparatus 120 executes (starts) the control making the parasympathetic nervous system dominant in the time zone in which the parasympathetic nervous system should be made dominant. FIG. 6 is a flow chart in operation example 1.

Controller 121 obtains a current time that is measured by time counter 123 (S11), and determines whether the obtained current time is a start time (S12). The start time is, for example, 8:00 p.m., and is preset, but may be set through a setting operation by subject 200 received by operation receiver 125.

When determining that the current time is the start time (Yes in S12), controller 121 executes the control making the parasympathetic nervous system dominant (S13). When determining that the current time is not the start time (No in S12), controller 121 keeps obtaining a current time (S11) and determining whether the current time is the start time (S12).

In this way, environmental control system 10 obtains current time information, and executes the control making the parasympathetic nervous system dominant based on the obtained current time information. Environmental control system 10 is capable of executing (starting) the control making the parasympathetic nervous system dominant in the time zone in which the parasympathetic nervous system should be made dominant. In other words, it is possible to reduce the disorder of the autonomic nervous system of subject 200.

### [Operation Example 2]

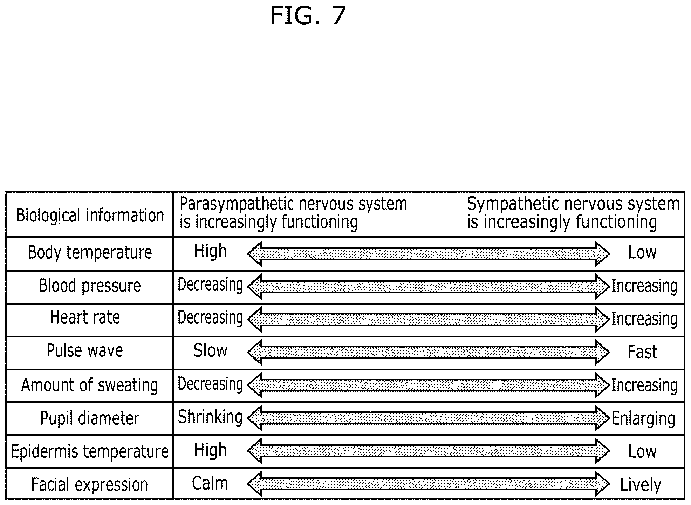

Control apparatus 120 may obtain biological information of subject 200, and execute the control based on the obtained biological information. The biological information is measured by biological information measuring apparatus 110. FIG. 7 is a diagram illustrating a relationship between the function of the sympathetic nervous system and the function of the parasympathetic nervous system, and change in biological information. As indicated in FIG. 7, the biological information of subject 200 relates to the function of the parasympathetic nervous system. The biological information includes, for example, measured data of body temperatures, blood pressures, heart rates, pulse waves, the amounts of sweating, pupil diameters, epidermis temperatures, and facial expressions, etc. The measured data of the biological information can be used as indicators for starting the control making the parasympathetic nervous system dominant.

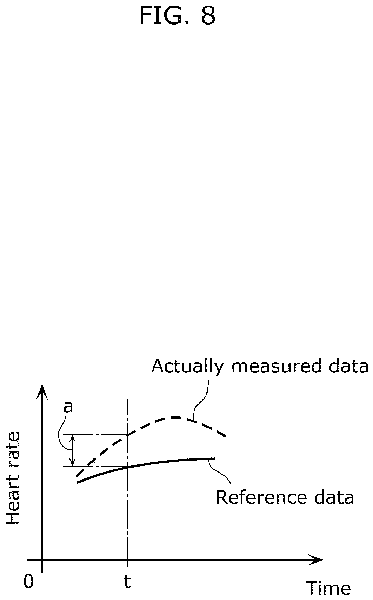

FIG. 8 is a diagram for explaining execution timings of control based on heart rates. For example, changes in heart rate in resting periods in several days of subject 200 are obtained, and the average change in heart rate per day is stored as reference data of the heart rates onto storage 124. The reference data is indicated by a solid line in FIG. 8. Although the reference data is optimized for subject 200, it is to be noted that absolute reference data to be applied to any person can be used instead of the reference data.

Controller 121 monitors the heart rate of subject 200 measured by biological information measuring apparatus 110, and compares the heart rate with reference data stored in storage 124. When the heart rate is higher than the reference data, the function of the parasympathetic nervous system is estimated to be weak. Thus, it is considered that the control making the parasympathetic nervous system dominant needs to be performed.

In view of this, for example, controller 121 executes (starts) the control making the parasympathetic nervous system dominant at time t as a predetermined timing at which the heart rate that is measured by biological information measuring apparatus 110 becomes larger than the reference data by threshold value a (a > 0). When the function of the parasympathetic nervous system of subject 200 is estimated to be weaker than normal, environmental control system 10 is capable of executing the control making the parasympathetic nervous system dominant. In other words, it is possible to reduce the disorder of the autonomic nervous system of subject 200. It is to be noted that the same operation can be performed using biological information other than the heart rates.

### [Effects, etc.]

As described above, environmental control system 10 includes: air conditioner 30 for adjusting a temperature in space 300 in which subject 200 is located; and control apparatus 120 which performs control that makes a parasympathetic nervous system of subject 200 dominant over a parasympathetic nervous system of subject 200 by performing first control and second control using the air conditioner. The first control increases the temperature in space 300, and the second control cyclically changes the temperature in space 300 in such a manner that a temperature difference from a largest value to a smallest value in the space falls within 3 degrees Celsius.

Environmental control system 10 is capable of cyclically changing the temperature in space 300 slightly after providing an environment in which the temperature is rather warm in space 300, thereby being able to reduce stimuli by temperatures given to subject 200 and increase the comfortableness. This makes it possible to make the parasympathetic nervous system of subject 200 dominant. Environmental control system 10 makes the parasympathetic nervous system of subject 200 dominant, thereby being able to, for example, reduce the feeling of nervousness of subject 200 and increase the feeling of relaxing of subject 200.

In addition, environmental control system 10 includes: ventilator 70 which ventilates space 300 in which subject 200 is located; and control apparatus 120 which performs control that makes a parasympathetic nervous system of subject 200 dominant over a sympathetic nervous system of subject 200 by setting a concentration of carbon dioxide in space 300 to 1000 ppm or more using ventilator 70.

Environmental control system 10 adjusts the concentration of carbon dioxide in space 300 to 1000 ppm or more, thereby being able to make the parasympathetic nervous system of subject 200 dominant. Environmental control system 10 makes the parasympathetic nervous system of subject 200 dominant, thereby being able to, for example, reduce the feeling of nervousness of subject 200 and increase the feeling of relaxing of subject 200.

In addition, for example, environmental control system 10 further includes air conditioner 30 for adjusting a temperature in space 300. In the control, control apparatus 120 performs first control and second control using air conditioner 30. The first control increases the temperature in space 300, and second control cyclically changes the temperature in space 300 in such a manner that a temperature difference from a largest value to a smallest value falls within 3 degrees Celsius.

Environmental control system 10 is capable of cyclically changing the temperature in space 300 slightly after providing an environment in which the temperature is rather warm in space 300, thereby being able to reduce stimuli by temperatures given to subject 200 and increase the comfortableness. This makes it possible to make the parasympathetic nervous system of subject 200 dominant.

In addition, for example, the first control is control of increasing the temperature in space 300 using air conditioner 30 for a predetermined period of time, and the second control is control of cyclically changing the temperature in space 300 using air conditioner 30 at a cycle different from the predetermined period of time in such a manner that the temperature difference from a largest value to a smallest value falls within 3 degrees Celsius.

Environmental control system 10 is capable of cyclically changing the temperature in space 300 slightly after providing an environment in which the temperature is rather warm in space 300, thereby being able to reduce stimuli by temperatures given to subject 200 and increase the comfortableness. This makes it possible to make the parasympathetic nervous system of subject 200 dominant.

In addition, for example, environmental control system 10 further includes lighting apparatus 40 which illuminates space 300. Control apparatus 120 decreases an illuminance in space 300 using lighting apparatus 40 in an initial period from a start of the control.

Environmental control system 10 is capable of making the parasympathetic nervous system dominant by weakening the stimuli by light given to subject 200 by reducing the illuminance in space 300.

In addition, for example, environmental control system 10 further includes lighting apparatus 40 which illuminates space 300. Control apparatus 120 decreases a color temperature of light that is emitted by lighting apparatus 40 in an initial period from a start of the control.

Environmental control system 10 is capable of making the parasympathetic nervous system of subject 200 dominant by providing space 300 with the illuminance environment in which the color temperature is low.

In addition, for example, environmental control system 10 further includes outside light adjusting apparatus 50 which adjusts a light amount of outside light that enters space 300. Control apparatus 120 decreases the light amount of the outside light that enters space 300 using outside light adjusting apparatus 50 in an initial period from a start of the control.

Environmental control system 10 is capable of making the parasympathetic nervous system of subject 200 dominant by weakening the stimuli by light given to subject 200 by reducing the illuminance in space 300.

In addition, for example, environmental control system 10 further includes indirect lighting apparatus 60 disposed in space 300. In the control, control apparatus 120 fluctuates an illuminance of light that is emitted by indirect lighting apparatus 60.

Environmental control system 10 is capable of making the parasympathetic nervous system of subject 200 dominant.

In addition, for example, environmental control system 10 further includes indirect lighting apparatus 60 disposed in space 300. In the control, control apparatus 120 is capable of changing an emission color of light that is emitted by indirect lighting apparatus 60.

Environmental control system 10 is capable of making the parasympathetic nervous system of subject 200 dominant by causing indirect lighting apparatus 60 to emit light having a blue-tone color (or green-tone color).

In addition, for example, environmental control system 10 further includes speaker 80 and scent generator 90 which are disposed in space 300. In the control, control apparatus 120 changes a sound that is output by speaker 80 and changes a scent that is generated by scent generator 90.

Environmental control system 10 is capable of making the parasympathetic nervous system of subject 200 dominant by causing a sound having a relaxing effect to be output and causing a scent having a relaxing effect to be generated.

In addition, for example, environmental control system 10 further includes environment measuring apparatus 100 which measures environmental information in space 300 in which subject 200 is located. Environment measuring apparatus 100 is one example of a measuring apparatus.

Environmental control system 10 is capable of performing the control based on the environment in space 300.

In addition, control apparatus 120 obtains current time information, and executes the control based on the current time information obtained.

Environmental control system 10 is capable of executing the control making the parasympathetic nervous system dominant in the time zone in which the parasympathetic nervous system should be made dominant. In other words, it is possible to reduce the disorder of the autonomic nervous system of subject 200.

In addition, control apparatus 120 obtains biological information of subject 200, and executes the control based on the biological information obtained.

When the function of the parasympathetic nervous system of subject 200 is estimated to be weaker than normal based on the biological information, environmental control system 10 is capable of executing the control making the parasympathetic nervous system dominant. In other words, it is possible to reduce the disorder of the autonomic nervous system of subject 200.

In addition, an environmental control method that is executed by a computer such as environmental control system 10 includes performing control that makes a parasympathetic nervous system of subject 200 dominant over a parasympathetic nervous system of subject 200 using air conditioner 30 for adjusting a temperature in a space in which subject 200 is located, by increasing the temperature in space 300 and cyclically changing the temperature in space 300 in such a manner that a temperature difference from a largest value to a smallest value in space 300 falls within 3 degrees Celsius.

The environmental control method makes it possible to cyclically change the temperature in space 300 slightly after providing an environment in which the temperature is rather warm in space 300, thereby making it possible to reduce stimuli by temperatures given to subject 200 and increase the comfortableness. This makes it possible to make the parasympathetic nervous system of subject 200 dominant. The environmental control method makes it possible to, for example, reduce the feeling of nervousness of subject 200 and increase the feeling of relaxing of subject 200 by making the parasympathetic nervous system of subject 200 dominant.

In addition, another environmental control method that is executed by a computer such as environmental control system 10 includes performing control that makes a parasympathetic nervous system of subject 200 dominant over a sympathetic nervous system of subject 200 by setting a concentration of carbon dioxide in space 300 to 1000 ppm or more using ventilator 300 which ventilates space 300 in which subject 200 is located.

The environmental control method makes it possible to make the parasympathetic nervous system of subject 200 dominant by adjusting the concentration of carbon dioxide in space 300 to 1000 ppm or more. The environmental control method makes it possible to, for example, reduce the feeling of nervousness of subject 200 and increase the feeling of relaxing of subject 200 by making the parasympathetic nervous system of subject 200 dominant.

### [Other Embodiments]

Although the embodiment has been described above, the present invention is not limited to the above embodiment.

For example, in the above embodiment, the processing executed by a particular processing unit may be executed by another processing unit. The order of a plurality of processes may be changed, or a plurality of processes may be executed in parallel.

In the above embodiment, each of the constituent elements may be implemented by a software program suitable for the constituent element being executed. Each of the constituent elements may be implemented by means of a program executer such as a CPU or a processor reading and executing a software program recorded on a recording medium such as hard disc or semiconductor memory.

In addition, each of the constituent elements may be executed by hardware. Each of the constituent elements may be a circuit (or an integrated circuit). These circuits may be configured as a single circuit as a whole, or may be configured as individual circuits. In addition, these circuits may be general-purpose circuits, or dedicated circuits.

Alternatively, the general or specific embodiment of the present invention may be implemented as a system, an apparatus, a method, an integrated circuit, a computer program, or a recording medium such as a computer-readable CD-ROM. Alternatively, the general or specific embodiment of the present invention may be implemented as a combination of a system, an apparatus, a method, an integrated circuit, a computer program, or a recording medium.

For example, the present invention may be implemented as an environmental control method, a program for causing a computer to execute the environmental control method, or a non-transitory computer-readable recording medium on which such a program is recorded.

Alternatively, the present invention may be implemented as a control apparatus according to the embodiment, or as a program which is executed by a computer in order to cause the computer to function as such a control apparatus. Alternatively, the present invention may be implemented as a computer-readable non-transitory recording medium on which such a program is recorded.

In addition, the environmental control system is implemented as a plurality of apparatuses in the embodiment, but may be implemented as a single apparatus. When the environmental control system is implemented as a plurality of apparatuses, the constituent elements of the environmental control system described in the embodiment may be allocated to a plurality of apparatuses in any way.

Furthermore, the present invention encompasses embodiments obtainable by adding, to any of these embodiments, various kinds of modifications that a person skilled in the art would arrive at and embodiments configurable by combining constituent elements in different embodiments without deviating from the scope of the present disclosure.

### [Reference Signs List]

10 environmental control system
20 wind blower
30 air conditioner
40 lighting apparatus
50 outside light adjusting apparatus
60 indirect lighting apparatus
70 ventilator
80 speaker
90 scent generator
100 environment measuring apparatus (measuring apparatus)
120 control apparatus
200 subject
300 space

## Claims

1. An environmental control system, comprising:
an air conditioner for adjusting a temperature in a space in which a subject is located; and
a control apparatus which performs control that makes a parasympathetic nervous system of the subject dominant over a parasympathetic nervous system of the subject by performing first control and second control using the air conditioner, the first control increasing the temperature in the space, the second control cyclically changing the temperature in the space in such a manner that a temperature difference from a largest value to a smallest value in the space falls within 3 degrees Celsius.

2. An environmental control system, comprising:
a ventilator which ventilates a space in which a subject is located; and
a control apparatus which performs control that makes a parasympathetic nervous system of the subject dominant over a sympathetic nervous system of the subject by setting a concentration of carbon dioxide in the space to 1000 ppm or more using the ventilator.

3. The environmental control system according to claim 2, further comprising:
an air conditioner for adjusting a temperature in the space,
wherein, in the control, the control apparatus performs first control and second control using the air conditioner, the first control increasing the temperature in the space, the second control cyclically changing the temperature in the space in such a manner that a temperature difference from a largest value to a smallest value in the space falls within 3 degrees Celsius.

4. The environmental control system according to claim 1 or claim 3,
wherein the first control is control of increasing the temperature in the space using the air conditioner for a predetermined period of time, and
the second control is control of cyclically changing the temperature in the space using the air conditioner at a cycle different from the predetermined period of time in such a manner that the temperature difference from a largest value to a smallest value falls within 3 degrees Celsius.

5. The environmental control system according to any one of claim 1 to claim 4, further comprising:
a lighting apparatus which illuminates the space,
wherein the control apparatus decreases an illuminance in the space using the lighting apparatus in an initial period from a start of the control.

6. The environmental control system according to any one of claim 1 to claim 5, further comprising:
a lighting apparatus which illuminates the space,
wherein the control apparatus decreases a color temperature of light that is emitted by the lighting apparatus in an initial period from a start of the control.

7. The environmental control system according to any one of claim 1 to claim 6, further comprising:
an outside light adjusting apparatus which adjusts a light amount of outside light that enters the space,
wherein the control apparatus decreases the light amount of the outside light that enters the space using the outside light adjusting apparatus in an initial period from a start of the control.

8. The environmental control system according to any one of claim 1 to claim 7, further comprising:
an indirect lighting apparatus disposed in the space,
wherein, in the control, the control apparatus fluctuates an illuminance of light that is emitted by the indirect lighting apparatus.

9. The environmental control system according to any one of claim 1 to claim 8, further comprising:
an indirect lighting apparatus disposed in the space,
wherein, in the control, the control apparatus is capable of changing an emission color of light that is emitted by the indirect lighting apparatus.

10. The environmental control system according to any one of claim 1 to claim 9, further comprising:
a speaker and a scent generator which are disposed in the space,
wherein, in the control, the control apparatus changes a sound that is output by the speaker, and changes a scent that is generated by the scent generator.

11. The environmental control system according to any one of claim 1 to claim 10, further comprising:
a measuring apparatus which measures environmental information in the space.

12. The environmental control system according to any one of claim 1 to claim 11,
wherein the control apparatus obtains current time information, and executes the control based on the current time information obtained.

13. The environmental control system according to any one of claim 1 to claim 12,
wherein the control apparatus obtains biological information of the subject, and executes the control based on the biological information obtained.

14. An environmental control method, comprising:
performing control that makes a parasympathetic nervous system of the subject dominant over a parasympathetic nervous system of the subject using an air conditioner for adjusting a temperature in a space in which a subject is located, by increasing the temperature in the space and cyclically changing the temperature in the space in such a manner that a temperature difference from a largest value to a smallest value in the space falls within 3 degrees Celsius.

15. An environmental control method, comprising:
performing control that makes a parasympathetic nervous system of the subject dominant over a sympathetic nervous system of the subject by setting a concentration of carbon dioxide in the space to 1000 ppm or more using a ventilator which ventilates a space in which a subject is located.
